# EUROPEAN PATENT APPLICATION

(11) **EP 4 015 013 A1**
(43) Date of publication of application: **22.06.2022**
(21) Application number: 20214462.2
(22) Date of filing: 16.12.2020
(51) Int. Cl.: A61M 1/16, A61M 1/28

(54) **PERITONEAL DIALYSIS SYSTEM AND A CONTROL SYSTEM THEREFOR**

(71) Applicant: Bellco S.r.l. con Unico Socio, 41037 Modena (IT)
(72) Inventor: MARRA, Antonio Giuseppe, 41037 Modena (IT); VESPERINI, Genni, 41037 Modena (IT); SICIGNANO, Luca, 41037 Modena (IT)
(74) Representative: Maschio, Antonio

(57) **Abstract**

A water purification unit (30) fluidly connectable to a peritoneal dialysis preparator (50/60) and a cycler. The purification unit is connectable to a water source (20) and has a pump for flow of fluid from the unit to a purified water reservoir (40), the reservoir being fluidly connectable to the preparator which has a number of solute containers for dissolution of the solutes; each container being fluidly connectable to a chamber for receiving a solution of the solutes for preparation of a peritoneal dialysis fluid and the chamber being fluidly connectable to the cycler for the delivery and drainage of the peritoneal dialysis fluid to and from a patient. Purified water delivery to the reservoir is controlled by a first pressure sensor (72), and a second pressure sensor (74) between the reservoir and preparator provides a pressure differential between the first and second sensors to control a flow generator (76).

## Description

### FIELD

The present invention relates to a peritoneal dialysis system and in particular to improved control of a peritoneal dialysis system.

### BACKGROUND

One type of dialysis for the treatment of kidney failure is peritoneal dialysis (PD) which uses the lining of a patient's abdomen, the peritoneum, to filter blood while its remains inside the body. Peritoneal Dialysis is an effective way to treat patients with End Stage Renal Disease and acute patients with renal failure. The patient is provided with a catheter that includes a transfer set for selective connection to a dialysis solution or a drain bag. A dialysis solution ('dialysate' or 'peritoneal dialysis fluid') comprising water with other electrolytes is warmed to body temperature and then introduced into the peritoneal cavity of the patient via the catheter. The solution remains here for a period of time required to clean the blood of waste products, toxins and excess fluid and these are then removed from the body to leave the right amounts of electrolytes and nutrients in the blood. The machine or 'cycler' may repeat the exchange process a number of times (cycles), as required for a particular patient.

Conventionally dialysate consists of purified water, glucose and electrolytes, with the concentration of electrolytes resembling that which occurs naturally in the blood. It is prepared according to an individual patient's needs to help regulate their electrolyte and acid-base balance and remove metabolic waste products. The dialysate components are normally shipped in the form of concentrates, with basic and acidic concentrates being prepared separately. The basic concentrate is usually supplied as a dry concentrate consisting of pure sodium bicarbonate which is made up into a saturated solution and the acidic concentrate is usually provided as a liquid concentrate. If it is provided as a dry concentrate, the entire concentrate must be dissolved before being diluted to form the dialysate. This requires a large volume of liquid and multiple components for providing the peritoneal dialysis fluid, increasing the time required for production of the fluid. Concentrate may also be wasted during the production of the correct peritoneal dialysis fluid and difficulties may be encountered in providing the correct concentration of electrolytes in the fluid for a particular patient. Thus, while it is preferable to provide the concentrates in dry form to reduce their volume for shipping and storage purposes, providing the correct dialysate for a particular patient on a continuous basis can prove problematic. As a result, multiple bags of PDF are normally provided pre-prepared for dialysis. The high weights of peritoneal dialysis fluid (PDF) bags reduces the appeal of this procedure, as does the number, volume and weight of supplies required for each daily treatment (up to 15-18 Liters every day).

It is an object of the present invention to provide an improved peritoneal dialysis system that aims to overcome or at least alleviate the above-mentioned drawbacks.

### SUMMARY

A first aspect of the present invention provides a peritoneal dialysis system comprising a water purification unit fluidly connectable to a peritoneal dialysis preparator and cycler, the purification unit being fluidly connectable to a water source and including at least one pump for flow of fluid from the unit to a purified water reservoir, the reservoir being fluidly connectable to the preparator, the preparator comprising a plurality of containers each containing one or more solutes for dissolution of the solutes; each container being fluidly connectable to a chamber for receiving a solution of the solutes for preparation of a peritoneal dialysis fluid, said chamber being fluidly connectable to the cycler for the delivery and drainage of the peritoneal dialysis fluid to and from a patient, wherein at least one hydraulic flow generator is provided in at least one of the fluid paths between the water source and the containers, the containers and the chamber and the chamber and the cycler and wherein at least one pressure sensor is provided between the water purification unit and the reservoir.

The at least one pressure sensor controls filling of the reservoir, preventing overfilling thereof. More preferably, a second pressure sensor is provided between the reservoir and the preparator. The at least one hydraulic flow generator is preferably provided between the reservoir and the second pressure sensor, the second pressure sensor preferably being provided at an outflow of the flow generator. In a preferred embodiment, a pressure differential between the first and second pressure sensors is detected wherein detection of a predetermined pressure differential activates or deactivates the flow generator. Preferably, a substantially equal flow pressure before and after the flow generator represents normal flow. Feedback from the second pressure sensor compares the actual output with a desired output in a closed loop system whereby detection of predetermined minimum pressure differential between the first and second sensors deactivates flow, for example by stopping the flow generator or cinching a fluid flow to the preparator.

Preferably, the pressure sensor outputs are transmitted to a microprocessor, preferably provided as part of the dialysis system, such as within a controller provided in a dialysis machine that houses the fluid system. The microprocessor may be programmed to monitor the fluid pressure at the first and/or second sensors and/or monitor the difference in the fluid pressure between the two, wherein upon detection of a predetermined pressure differential, the flow generator upstream of the second sensor is activated or deactivated.

The water purification unit may purify water taken from any source, such as mains water from a domestic tap or faucet. The purification unit preferably incorporates a semi-permeable membrane, such as a reverse osmosis membrane, for purifying the water.

A second aspect of the present invention provides a water purification unit for fluid connection to a peritoneal dialysis preparator and cycler, the purification unit being fluidly connectable to a water source, the unit comprising at least one vessel having a semi-permeable membrane, at least one pump and at least one purified water reservoir in fluid communication with the vessel, the reservoir being fluidly connectable to a dialysis preparator and cycler, wherein a hydraulic flow generator is provided in at least one of fluid path between the vessel and the preparator and at least one pressure sensor is provided between the vessel and the reservoir.

More preferably, the flow generator is provided between the reservoir and the preparator and the pressure sensor is provided between the vessel and the reservoir. More preferably still, a second pressure sensor is provided between the reservoir and the preparator, at an outlet of the flow generator.

Preferably, the system according to the first aspect of the invention is further provided with an arrangement of hydraulic flow or pressure generators throughout the other fluid paths to provide optimized dissolution of the solutes, optimized mixing of the solution and/or cycling of the peritoneal dialysis fluid thus formed. It is preferable for at least one of the hydraulic flow generators to be a variable flow generator. The different configurations of hydraulic flow/pressure generators are provided in the fluid paths to provide optimization of the flow of water through the fluid system to create the correct powder concentrates and subsequent peritoneal dialysis fluid for cycling.

Any suitable hydraulic flow generator may be provided, such as an actuator operable on the fluid line. A pump, such as a hydraulic piston pump or a peristaltic pump may form the hydraulic flow generator. The hydraulic flow generators may be provided within tubing extending between the component parts of the system but more preferably are included in a cassette operable within the dialysis system.

The cassette may be provided with other components that are conventional in the art and therefore will not be discussed here in any detail. Such components include, *inter alia,* a casing surrounding the fluid paths, pump regions, for example, being formed by chambers open to one side that are covered by a flexible plastic film and valve regions that may block the fluid paths. The cassette may also be provided with a heating region, such as heating elements provided in a part of the cassette that surrounds a fluid path. Sensors may also be provided within the cassette to monitor fluid flow and properties, such as temperature.

It is to be appreciated that the inlets and outlets of the cassette may be connected to other fluid paths by hose elements.

The at least one container is preferably a bag that contains one or more solutes, preferably in a concentrated form and provided in separate compartments. These concentrated solutes may for example be in the form of powder, pellets, super concentrated liquid and/or gel.

The bag preferably includes a nozzle attachment to aid mixing of the solute with the purified water.

### Brief description of the figures

**Figure 1** is a schematic drawing illustrating an automated peritoneal dialysis machine according to the prior art;
**Figure 2** is a block diagram of an automated peritoneal dialysis preparator and cycler machine for use with the present invention; and
**Figure 3** is a schematic diagram of a control system according to an embodiment of the present invention for use with the automated peritoneal dialysis preparator and cycler machine shown in Figure 2.

### DETAILED DESCRIPTION

### Definitions

Unless defined otherwise, all technical and scientific terms used generally have the same meaning as commonly understood by one of ordinary skill in the art.

The articles "a" and "an" are used to refer to one or to over one (*i.e.,* to at least one) of the grammatical object of the article. For example, "an element" means one element or over one element.

The term "bag" as used herein refers to a container, and preferably a flexible, collapsible and/or foldable container. Preferably a bag is a container for fluids, most preferably for liquids.

The term "comprising" includes, but is not limited to, whatever follows the word "comprising." Use of the term indicates the listed elements are required or mandatory but that other elements are optional and may be present.

The term "concentrated" as used herein means, of a substance or solution, present in a high proportion relative to other substances, preferably having had fluid, such as water or other diluting agents, removed or reduced.

The term "consisting of" includes and is limited to whatever follows the phrase "consisting of." The phrase indicates the limited elements are required or mandatory and that no other elements may be present.

The term "consisting essentially of" includes whatever follows the term "consisting essentially of" and additional elements, structures, acts or features that do not affect the basic operation of the apparatus, structure or method described.

The term "dialysate" describes a fluid into or out of which solutes from a fluid to be dialyzed diffuse through a membrane. An initial dialysate used for therapy typically contains electrolytes close in concentration to the physiological concentration of electrolytes found in blood. However, the concentration of the dialysate can change over the course of therapy, and can further be adjusted as desired.

The term "dialysis flow path" refers to a fluid pathway or passageway configured to convey a fluid, such as dialysate and/or blood, wherein said pathway forms at least part of, preferably the whole of, a fluid circuit for peritoneal dialysis, hemodialysis, hemofiltration, hemodiafiltration or ultrafiltration. A dialysis machine may be included within the flow path. A dialyzer may be included in the flow path.

The term "dissolution" as used herein refers to the process of a solute becoming incorporated into a solvent to make a solution. Dissolution means the same as "dissolving" and the terms are used interchangeably herein.

The term "dissolution time" as used herein refers to the time taken for one or more solutes to fully dissolve in a solvent to make solution. Dissolution times of the same solutes can be compared if the conditions of dissolution (including for example solvent type and volume, temperature, flow speed of solvent and container shape) are the same.

The term "fluid" can be any substance without a fixed shape that yields easily to external pressure such as a gas or a liquid. Specifically, the fluid can be water, preferably purified water, and can be water containing any solutes at any concentration. The fluid can be used as a solvent (to dissolve one or more solutes). The fluid can also be dialysate of any type including fresh, partially used, or spent. The fluid can also contain one or more dissolved gas. The fluid may be blood.

The term "fluidly connectable" refers to the ability to provide passage of fluid from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Most preferably the connections are provided by tubes or pipes.

The term "fluidly connected" refers to a particular state or configuration of one or more components such that fluid can flow from one point to another point. The connection state can also include an optional unconnected state or configuration, such that the two points are disconnected from each other to discontinue flow. It will be further understood that the two "fluidly connectable" points, as defined above, can from a "fluidly connected" state. The two points can be within or between any one or more of compartments, modules, systems, components, and rechargers, all of any type.

The term "formed" or "formed of" as used herein refers to a material comprising any of the materials, elements, composited or compounds listed.

The term "inlet" can refer to a portion of a component through which fluid and/or gas can be drawn into a component, conduit, or fluid passageway of any type, such as a bag, container, conduit, tube or pipe of any type.

The term "measuring" or "to measure" can refer to determining any parameter or variable. The parameter or variable can relate to any state or value of a system, component, fluid, gas, or mixtures of one or more gases or fluids.

The term "measurement" refers to data or information of the parameter or variable determined.

The term "mix" means to combine one or more substance so that the resulting mixture is not easily separated. Mixing, especially evenly spreading, of solute and solvent aids and speeds the process of dissolution. Mixing can be achieved by any method, including spraying, stirring, shaking or otherwise agitating. The term "improved mixing" refers to a situation wherein the components of a mixture are more evenly distributed and not clumped together. Improved mixing may be achieved, for example, by speeding the mixing up. In the context of a solution, improved mixing results in a solution of the correct concentration because all or most of the solutes dissolve, rather than remaining as clumps or aggregated within the solvent.

The term "nozzle" as used herein refers to a fitting, usually at the end of a tube or pipe, which controls the flow of a fluid (liquid or gas) through said fitting. It may control (in speed, direction or otherwise) the entry of the fluid into a container, especially a bag. It may accelerate or decelerate the fluid. It may control the direction of the fluid. The nozzle may control the density of spray of the fluid and direction of spray of fluid from the nozzle.

The term "outlet" refers to a portion of a component through which fluid or gas can be pulled out of said component and into another component, conduit, or fluid passageway of any type.

The term "pass through" refers to an action of fluid or gas passing a component positioned in a flow path. The fluid or gas can enter an inlet of the component and exit via an outlet of the component to continue the flow path.

The term "pressure" refers to a force exerted by a gas or liquid on the walls of a component, container, or conduit.

"Purified water" can be defined as water produced by distillation, deionization, reverse osmosis, or other suitable processes that remove impurities in water.

The term "reconstituting" or "re-constituting as used herein refers to restore to an original state. In the context of the solutions of the invention, this term is used to mean returning a solution to its original form, before liquids were removed, or in creating a solution from concentrated liquid and/or dried components.

The term "reconstituted" or "re-constituted" refers to an item, product or solution produced after reconstituting as defined above.

The term "solute" as used herein refers to a substance dissolved in a solution. The solute may comprise one or more chemicals or compounds. Solutes as used herein, before being dissolved in a solvent, may exist as entirely or partially dried, gels, concentrated liquids, pellets or powders, or any combination of these forms.

The term "solution" as used herein refers to a liquid mixture in which a solute is uniformly distributed within a solvent. A "solution for dialysis" may refer to any solution which can be used in any form of dialysis. It is envisaged that the solutions described herein contain specific concentrations of solutes.

The term "solvent" as used herein refers to a substance which dissolves a solute. Preferably the solvent is a liquid, and most preferably it is water.

As used herein, the "Venturi effect" reduction in fluid pressure, and increase in velocity, caused by a fluid flowing through a constricted section of a tube or pipe. Preferably the constriction is caused by a reduction in diameter of the tube or pipe.

The term "water purification unit" refers to any single component or system which can produce purified water (as defined above) from tap water or other water containing any type of impurity.

The term "upstream" refers to a fluid path towards or nearer to the water source, furthest from the cycler.

The term "downstream" refers to a fluid path towards or nearer the cycler, further from the water source.

### System

The present invention relates to an improved automated peritoneal dialysis machine that includes a preparator and cycler for *in situ* preparation of peritoneal dialysis fluid (PDF) for delivery and drainage from a patient and, in particular to a control system for such a dialysis system.

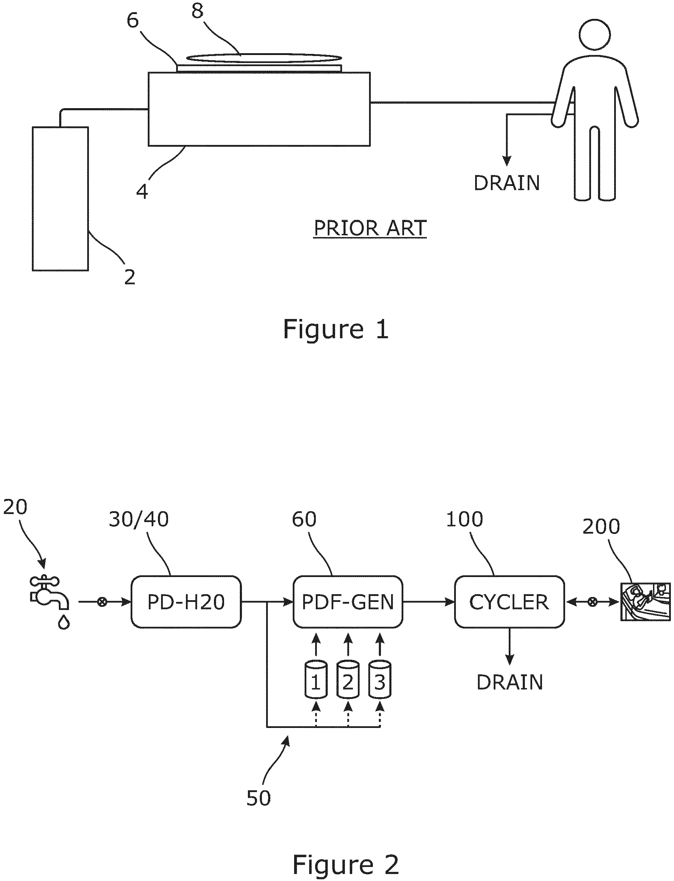

The basic concept of an automated peritoneal dialysis machine according to the prior art is shown in FIG. 1 of the accompanying drawings. The machine is fluidly connected to a patient via a catheter and transfer set and the machine has a container or bag 2 with a fresh solution supply of peritoneal dialysis fluid (PDF) which is delivered to a heating bag 8 placed on a heat source, such as a heating plate 6 to warm the PDF prior to delivery to the patient. The fluid remains in the patient for a predetermined dwell time and then removed from the patient, normally being collected in a drainage bag. The parts are fluidly connectable to provide fluid paths through the system creating a fluid system which is controlled by a controller 4. The machine includes a pump for transport of the fluid to and away from the patient, valves to control the fluid flows, a heat source to warm the PDF prior to its introduction into the patient and sensors connected to a controller to monitor and control the process.

The coupling of the fluid paths to the machine may be conveniently achieved by the provision of a disposable cassette (not shown) for receipt within a cassette holder of the machine which includes connections to each fluid path. The cassette provides sterile, disposable fluid pathways and generally includes a pump region, valve region, a sensor region and optionally a heating region. The cassette has connections for coupling with the dialysate container, the catheter unit and the drainage outflow.

The machine controls and monitors the introduction of fresh PDF into the abdominal cavity via the inlet and transfer set and the elimination of waste fluid via the transfer set and outlet. Sequential treatment cycles are carried out on a patient, normally overnight. However, the production of sufficient PDF for these cycles, particularly in a home-based setting, can be problematic.

The Applicant has devised an improved modified peritoneal dialysis machine that includes a preparator as well as a cycler to form the peritoneal dialysis system. Figure 2 of the accompanying drawings provides a high level block diagram of the preparator and cycler. The system delivers water, such as purified drinking water 40 from a tap 20 via a water purification system 30 such as reverse osmosis membrane, into one or more containers 1,2,3 collectively shown as 50 with different powders to create a concentrate and then moves this concentrate to a mixing bag to create the peritoneal dialysis fluid 60. Drinking water is supplied to a purification system and this purified water is then moved through powdered chemical constituents to create dissolution of the chemicals and proportioned to obtain the PDF solution for passing to the cycler 100. The powder concentrates may be provided in compartmentalized constituents bag with each compartment 1, 2, 3 having an inlet and outlet with a two-way valve whereby purified water can enter each compartment and is then delivered to a mixing bag 60 prior to delivery to the cycler. The cycler then delivers fresh PDF to the patient 200 and removes waste fluid via the drain outlet. This solves major problems on automated peritoneal diffusion, creating PDF starting from drinking water and containers with powders/concentrates, providing key benefits such as a reduction of the weight of the bags/containers that the patient has to handle; significant reduction of number, volume and weight of supplies; easier operation for patient; reduced infection risk; therapy customization and GDP reduction.

The combined peritoneal dialysis machine having a preparator as well as a cycler must be able to reconstitute powders (dissolve them in a liquid) and proportion them to create a pharma fluid suitable for delivery to a patient. Ideally, bags are provided that are equipped with nozzles to generate a turbulent flow, particularly using the Venturi effect, necessary to dissolve the powder and a hydraulic machine generates the flow to move different amounts of fluid. The system has a reconstitution part wherein the powders are dissolved and a proportioning part to proportion the right amount of concentrate formed from the dissolved powders.

The reconstitution part of the system includes the bags filled with the powders required to form the correct concentrate, such as dextrose, magnesium and calcium and lactate bicarbonate and sodium chloride. Electro valves are provided on entry and exit to the bags to deviate the flow path. A high pressure flow is required to ensure that the powders are completely dissolved, and a heater may be provided to aid dissolution and to raise the temperature of the resultant PDF. The proportioning part of the system requires a more accurate flow rate adjuster, such as a peristaltic pump, to move precise amounts of liquid in order to proportion the correct amount of concentrate, together with a scale system to measure the mass of the three solutions in order to proportion the right amount of concentrate.

However, one challenge with this system is to provide accurate control of fluid flow throughout the system using minimal component parts. It is necessary to control filling of the container 40 (generally in the form of a bag) to prevent over filling of the bag from the water purification system 30 and to control the flow of purified water to the concentrate containers 50 to the PDF mixing bag 60 to ensure that the correct proportion of purified water and concentrates are mixed to obtain the desired PDF, ideally being provided in a completely automated way. The system uses a volumetric approach to control the hydraulic flow paths that introduce purified water to the powder concentrates, provide mixing of the concentrate to form the PDF and delivery of the freshly made PDF to the patient. In particular, different configurations of hydraulic flow/pressure generators and/or pressure sensors are provided in the fluid paths to provide optimization of the flow of water from the water source and through the fluid system to create the correct powder concentrates and subsequent peritoneal dialysis fluid for cycling. The particular configurations may be provided within a disposable cassette having an inlet connection, an outlet connection, at least one connector to the pure water source and an a connector to each of the concentrate containers to enable accurate control of hydraulic flow paths through the preparator and cycler.

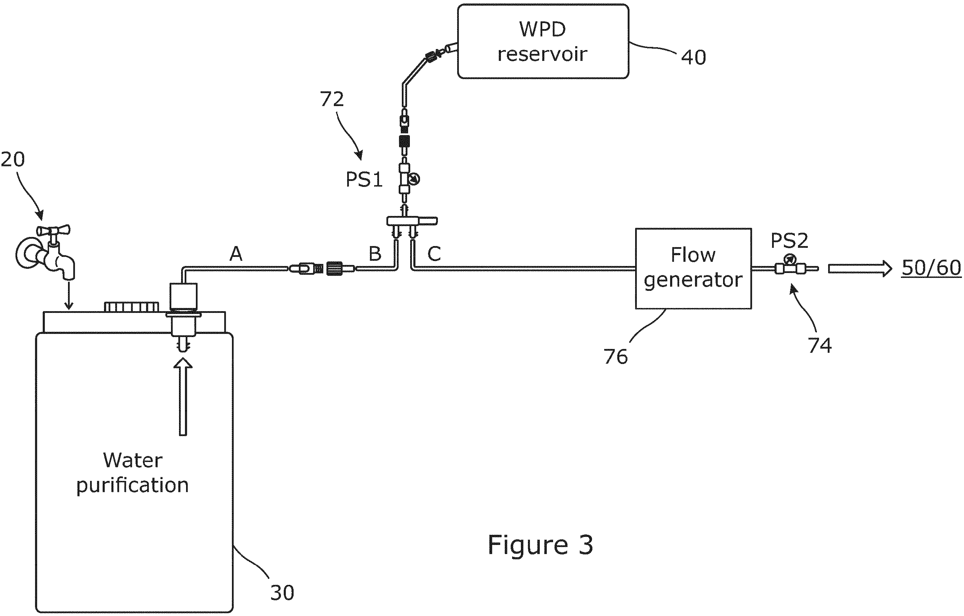

Figure 3 of the accompanying drawings illustrates one embodiment of a control system for the peritoneal dialysis preparator and cycler of the invention, the control system being provided between the inflow A, B of purified water to a purified water reservoir 40 and the outflow C of this water from the reservoir to the preparator 50, 60. A pressure sensor 72 is provided in the fluid path to the reservoir 40 and controls the pump provided in the water purification unit 30 and a second pressure sensor 74 is provided in the fluid path between the reservoir 40 and the preparator 50, 60 downstream of a flow generator 76. These two pressure sensors 72, 74 control the flow generator 76 by monitoring the difference in pressure between the first and second sensors 72, 74. This arrangement reduces the number of sensors in the hydraulic path, with the sensors acting as both control and protection sensors.

The pressure sensor outputs are transmitted to a microprocessor, preferably provided as part of the dialysis system, such as within a controller provided in a dialysis machine that houses the fluid pumping system. The microprocessor may be programmed to detect an unacceptable level of change in the fluid pressure on entry to the water bag which may signify overfilling of the bag and detect a predetermined pressure differential between the pressure of the bag and the fluid flow downstream of a flow generator 76 which activates or shuts down fluid flow to the preparator 50,60.

Thus, an example of sequence of events is as follows:

### Filling the reservoir

A pump is provided inside the water purification block for filling the reservoir 40 (electro-valve in B).

When the reservoir 40 is full and pressure sensor Ps1 (72) detects an over pressure to activate an Alarm to stop pump from filling reservoir.

### Filling the patient

Flow generator 76 has pressure sensor 74 with which can be read the patient pressure. The control loop is closed on this sensor to maintain the pressure under a predetermined value.

In this case, it is assumed that the pressure before and after the pump is equal and there is a relation in the memory of microcontroller that allows the change in pressure with the flow in relation of the tube dimension.

When the difference of the pressure between the first and second sensors exceeds a predetermined value, flow can be stopped, for example by cinching the tube.

Thus, the pressure sensors PS1 (72) and PS2 (74) permits control to prevent the overfill of the reservoir and to control flow via the flow generator within predetermined parameters.

The present invention provides an overall volume reduction in relation to the dialysis system due to the presence of reduced disposable component numbers. Furthermore, the system is more compact, providing cost savings with logistics and packaging and controlled via few component parts.

It is to be appreciated that all parts of the system should be leak-proof so that fluids cannot escape. The container or bags of the system described herein may be made of any material, preferably a polymer, most preferably a plastic. The bag may be rigid or flexible. Preferably the bag is flexible, resistant and stretchable, most preferably in any direction. The bag may be made of a multi-layered material, or a single layered material.

The system preferably uses a bag containing a v-shaped, funnel shaped or cone shaped section, with the opening for the nozzle at the point of the cone. Such a shape maximises the mixing of the fluid spray leaving the nozzle with the solutes in the bag to aid dissolution. However, the bag can be replaced by a solid container, preferably shaped with a cone-shaped part in which to fit a nozzle. Said container may be a box or carton, and is preferably made of a plastic.

Examples of solutes may for example comprise the following or any combination thereof:- all electrolytes, powdered acids and bases, citric acid, sodium chloride, hydrogen carbonate, calcium and magnesium ions and salts, sodium, potassium, citric acid, sodium bicarbonate and all bicarbonate salt forms, sodium lactate, and osmotic agents such as glucose, dextrose, polydextrose, polydextrine and xylitol. Dissolution of the solutes may create a medically useful

The dialysis system including optionally a cassette may include other conventional components of a dialysis machine, such as heating elements, controllers, sensors and scales. The system is particularly suitable for use in dialysis carried out at home, or another environment away from a hospital or medical practice. They may also be used in a hospital or any kind of medical practice.

One skilled in the art will understand that various combinations and/or modifications and variations can be made in the described apparatus, methods and uses depending upon the specific needs for operation. Moreover, features illustrated or described as being part of an aspect of the invention may be used in the aspect of the invention, either alone or in combination.

## Claims

1. A peritoneal dialysis system comprising a water purification unit fluidly connectable to a peritoneal dialysis preparator and a cycler, the purification unit being connectable to a water source and including at least one pump for flow of fluid from the unit to a purified water reservoir, the reservoir being fluidly connectable to the preparator, the preparator comprising a plurality of containers each containing one or more solutes for dissolution of the solutes; each container being fluidly connectable to a chamber for receiving a solution of the solutes for preparation of a peritoneal dialysis fluid, said chamber being fluidly connectable to the cycler for the delivery and drainage of the peritoneal dialysis fluid to and from a patient, wherein at least one hydraulic flow generator is provided in at least one of the fluid paths between the water source and the containers, the containers and the chamber and the chamber and the cycler, and wherein at least one pressure sensor is provided between the water purification unit and the reservoir.

2. The system as claimed in claim 1, wherein the at least one pressure sensor controls filling of the reservoir.

3. The system as claimed in claim 1 or claim 2, wherein a second pressure sensor is provided between the reservoir and the preparator.

4. The system as claimed in claim 3 wherein a hydraulic flow generator is provided between the reservoir and the second pressure sensor.

5. The system as claimed in claim 4 wherein the second pressure sensor is provided at an outflow of the flow generator.

6. The system as claimed in claim 4 or claim 5 wherein the system is configured to detect a pressure differential between the first and second pressure sensors whereby detection of a predetermined pressure differential activates or deactivates the flow generator.

7. The system as claimed in any one of the preceding claims wherein the pressure sensor outputs are transmitted to a microprocessor.

8. The system as claimed in any one of claims 4 to 7 further comprising providing a plurality of hydraulic flow or pressure generators throughout the other fluid paths to provide optimized dissolution of the solutes, optimized mixing of the solution and/or cycling of the peritoneal dialysis fluid.

9. The system as claimed in any one of claims 4 to 8 wherein the flow generator is as an actuator operable on the fluid line.

10. The system as claimed in claim 9 wherein the flow generator is selected from a hydraulic piston pump and a peristaltic pump.

11. The system as claimed in any one of the preceding claims further comprising a disposable dialysis cassette operable within the dialysis system.

12. A water purification unit for fluid connection to a peritoneal dialysis preparator and a cycler, the purification unit being fluidly connectable to a water source, the unit comprising at least one vessel having at least one semi-permeable membrane, at least one pump and at least one purified water reservoir in fluid communication with the vessel, the reservoir being fluidly connectable to a preparator and cycler, wherein a hydraulic flow generator is provided in at least one fluid path between the vessel and the preparator and at least one pressure sensor is provided between the water purification unit and the reservoir.

13. The unit of claim 12 wherein the at least one pressure sensor controls filling of the reservoir.

14. The unit as claimed in claim 12 or claim 13, wherein a second pressure sensor is provided between the reservoir and the preparator.

15. The unit as claimed in claim 14 wherein the hydraulic flow generator is provided between the reservoir and the second pressure sensor.
